(19) Europäisches Patentamt / European Patent Office / Office européen des brevets

(11) **EP 2 727 520 B1**

(12) **EUROPEAN PATENT SPECIFICATION**

(45) Date of publication and mention
of the grant of the patent:
**21.08.2019 Bulletin 2019/34**

(51) Int Cl.:
*A61B 5/00* *(2006.01)*    *G01N 33/68* *(2006.01)*
*G16H 50/20* *(2018.01)*   *G16B 40/00* *(2019.01)*

(21) Application number: **11868676.5**

(22) Date of filing: **08.12.2011**

(86) International application number:
**PCT/CN2011/083695**

(87) International publication number:
**WO 2013/000246 (03.01.2013 Gazette 2013/01)**

(54) **HEPATIC FIBROSIS DETECTION APPARATUS AND SYSTEM**

VORRICHTUNG UND SYSTEM ZUM NACHWEIS VON LEBERFIBROSE

APPAREIL ET SYSTÈME DE DÉTECTION DE LA FIBROSE HÉPATIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.06.2011 CN 201110173535
29.06.2011 CN 201120222828 U**

(43) Date of publication of application:
**07.05.2014 Bulletin 2014/19**

(73) Proprietor: **Inner Mongolia Furui Medical Science
Co., Ltd
Inner Mongolia 012000 (CN)**

(72) Inventors:
• **WANG, Guanyi
Beijing 100027 (CN)**
• **YANG, Yong
Beijing 100027 (CN)**
• **WANG, Xinhong
Bejing 100027 (CN)**

(74) Representative: **Lebkiri, Alexandre
Cabinet Camus Lebkiri
25, Rue de Maubeuge
75009 Paris (FR)**

(56) References cited:
**WO-A1-2007/051224    WO-A1-2010/097472**

WO-A1-2010/097472    WO-A1-2010/106140
WO-A1-2010/106140    CN-A- 101 023 356
CN-C- 100 416 273    US-A1- 2011 111 430
US-A1- 2011 111 430

• **SHAHEEN ABDEL AZIZ M ET AL: "FibroTest and
FibroScan for the prediction of hepatitis C-related
fibrosis: a systematic review of diagnostic test
accuracy",** AMERICAN JOURNAL OF
GASTROENTEROLOGY, ELSEVIER SCIENCE
INC, US, vol. 102, no. 11, 1 November 2007
(2007-11-01), pages 2589-2600, XP002563341,
ISSN: 0002-9270, DOI:
10.1111/J.1572-0241.2007.01466.X
• **ZHENG JIANG ET AL: "Support Vector
Machine-Based Feature Selection for
Classification of Liver Fibrosis Grade in Chronic
Hepatitis C",** JOURNAL OF MEDICAL SYSTEMS,
KLUWER ACADEMIC PUBLISHERS-PLENUM
PUBLISHERS, NE, vol. 30, no. 5, 12 September
2006 (2006-09-12), pages 389-394, XP019401048,
ISSN: 1573-689X, DOI:
10.1007/S10916-006-9023-2
• **KUSAKA K ET AL: "OBJECTIVE EVALUATION OF
LIVER CONSISTENCY TO ESTIMATE HEPATIC
FIBROSIS AND FUNCTIONAL RESERVE FOR
HEPATECTOMY",** JOURNAL OF THE AMERICAN
COLLEGE OF SURGEONS, COLLEGE,
CHICAGO, IL, US, vol. 191, no. 1, 1 January 2000
(2000-01-01), pages 47-53, XP002951815, ISSN:
1072-7515, DOI: 10.1016/S1072-7515(00)00309-4

**Description**

**Technical Field**

The present invention relates to the technical field of hepatic fibrosis research techniques, in particular, relates to hepatic fibrosis detection apparatus and system.

**Background Art**

At present, the clinical diagnosis of hepatic fibrosis and cirrhosis approximately includes the following categories: (1) Gold standard liver biopsy, i.e. hepatic fibrosis staging through pathology slide review after liver biopsy. In the commonly used methods, hepatitis B includes, for instance, 5 stages, namely S0, S1, S2, S3 and S4 (Chinese hepatitis B pathology scoring criteria), and hepatitis C, includes, for instance, 5 stages, namely F0, F1, F2, F3 and F4 (Metavir score). This method is an invasive diagnostic method. (2) Serum diagnosis: At present, there are more than 10 diagnostic models simulating serological variables. Such models obtain mathematical formula through mathematical calculation (such as statistical regression method) according to the combinations of different serological biochemical variables. (3) Image detection, such as ultrasonography, magnetic resonance (MR) imaging, and other imaging methods. (4) Ultrasonic elasticity imaging apparatus. For example, FibroScan (FS) measures the stiffness value of liver, and shows different stages by different range of values. This method can also be included in the scope of the image detection; (5) In addition, there is still emerging genetic testing, such as proteomics mapping. However, the gold standard liver biopsy is an invasive diagnostic method. It takes a long time for the patient to recover, has safety issues, and is affected by the sample deviation. Due to the reasons such as low accuracy and sensitivity or high cost, the existing serum biochemical marker model is not widely promoted and used in clinical diagnosis. The imaging method is limited by equipment. The stiffness value measured by FS is not only used for hepatic fibrosis detection, but also related to corresponding liver function and lesions to a certain extent. Fibroscan is promoted and applied, but is unable to be used to detect some patients because of its restrictions. The technical personnel in this field have always been striving to achieve the purpose of providing an easy-to-use and non-invasive method for diagnosis of hepatic fibrosis with high accuracy according to the actual situation.

The document WO 2010/106140 A1 entitled "Method for assessing liver fibrosis progression", the document WO 2010/097472 A1 entitled "Improved diagnosis of liver fibrosis or cirrhosis" and the document Zheng Jiang et al: «Support Vector Machine-Based Feature Selection for Classification of Liver Fibrosis Grade in Chronic Hepatitis C", Journal of Medical Systems, Kluwer Academic Publishers-plenum Publishers, NE, vol.30, no.5, 12 September 2006, pages 389-394 are known from the prior art."

Disclosure of the Invention

The objective of the present invention is to provide a hepatic fibrosis detection apparatus and system with improved detection accuracy, sensitivity and specificity, as defined in the appended claims.

Another objective of the present invention is to provide a hepatic fibrosis detection apparatus, comprising: an input device used to receive age and serum bio-chemical variables, where the serum biochemical variables at least comprise blood platelet, hyaluronic acid (HA), serum direct bilirubin (DBIL), prothrombin time (PT), serum glutamic pyruvic transaminase (ALT; GPT) and serum glutamic oxaloacetic transaminase (AST; GOT); a classifier used to perform hepatic fibrosis staging or inflammation diagnosis according to the age and serum biochemical variables received by the said input device; and an output device used to output the said hepatic fibrosis staging or inflammation diagnosis results of the said classifier.

The said serum biochemical variables further include serum alkaline phosphatase (ALP; AKP), serum cholinesterase (ChE) and prothrombin activity (PTA), or any one or two thereof.

The said serum biochemical variables also include the transforming growth factor β1 (TGF-β1) and α 2-macroglobulin (AMG);

The classifier is also used to receive transient elastography imaging data of the liver tissue for hepatic fibrosis staging according to the said age, said serum biochemical variables and said transient elastography imaging data of the liver tissue.

The said classifier includes the support vector machine classifier.

The said support vector machine classifier is a linear support vector machine classifier or a nonlinear classifier based on kernel method. Preferably, the said classifier further comprises a parameter trainer used to receive the training sample data and determine the parameters of the said classifier based on the said training sample data; wherein the said training sample data include at least the said age, serum biochemical variables and corresponding hepatic fibrosis staging. Training sample data may also include transient elastography imaging data.

Preferably, the said apparatus is realized in the form of a handheld device or a floor-standing device, an on-line diagnostic system, or a stand-alone computing device. Preferably, the apparatus also integrates a serum biochemical variable detection apparatus and/or a transient elastography imaging apparatus.

It is still another object of the present invention to provide a hepatic fibrosis detection system, including the above hepatic fibrosis detection apparatus and transient elastography imaging apparatus; wherein the said transient elastography imaging apparatus is used to obtain the transient elastic imaging data of the liver tissue; the said classifier receives transient elastography imaging data of the liver tissue from the said transient elastography imaging apparatus, and performs hepatic fibrosis staging according to the said age, said serum biochemical variables and said transient elastography imaging data of the liver tissue. Preferably, the system further comprises a serum biochemical variable detection apparatus.

The hepatic fibrosis detection apparatus and system in the present invention performs hepatic fibrosis staging in the light of the age and selected serum biochemical variables, and makes full use of various detection results, so that the hepatic fibrosis staging results are more accurate.

Further, the hepatic fibrosis detection apparatus and system in the present invention performs hepatic fibrosis staging in the light of the age, selected serum biochemical variables and transient elastography imaging data of the liver tissue, and makes full use of various detection results, so that the hepatic fibrosis staging results are more accurate.

Through the following detailed description of the exemplary embodiments of the present invention with reference to the appended drawings, other characteristics and advantages of the present invention will become clear.

**Brief Description of the Drawings**

Drawings composing a part of the Description are used to illustrate the embodiments of the present invention, and are used to explain the principle of the invention together with the Description.

The present invention can be more clearly understood with reference to the drawings and according to the following detailed description, where:

Figure 1 shows the structural diagram of a first embodiment of the hepatic fibrosis detection apparatus according to the present invention;
Figure 2 shows the structural diagram of a second embodiment of the hepatic fibrosis detection apparatus according to the present invention;
Figure 3 shows the structural diagram of a third embodiment of the hepatic fibrosis detection system according to the present invention;
Figure 4 shows the schematic view of an embodiment of the transient elastography imaging apparatus and the probe thereof;
Figure 5 shows the structural diagram of a fourth embodiment of the hepatic fibrosis detection system according to the present invention;
Figure 6 shows the structural diagram of a fifth embodiment of the hepatic fibrosis detection apparatus according to the present invention;
Figure 7 shows the schematic view of an example of the maximum margin SVM classification hyperplane;
Figure 8 shows the schematic view of an example of the nonlinear SVM algorithm.

**Detailed Embodiments of the Invention**

Here, various exemplary embodiments of the prevent invention are illustrated in detail with reference to the drawings. It should be noted that unless otherwise specified, the scope of the present invention is not limited to the relative layout, numerical expression and value of the components and steps illustrated in these embodiments.

At the same time, we should understand that in order to facilitate description, the size of each part shown in the appended drawings is not drawn in accordance with the actual proportional relation.

The following description of at least one exemplary embodiment is actually only illustrative, and is not intended to limit the present invention and its application or use under no circumstances. Technologies, methods, and devices known to general technical personnel in related fields may not be discussed in detail, but shall be regarded as part of the authorized description in proper cases.

In all the examples indicated and discussed here, any specific value should be interpreted as illustrative only, rather than restrictive. As a result, other examples of the illustrative embodiments may have different values. It should be noted that: similar numbers and letters show similar items in the appended drawings below. As a result, once an item is defined in a drawing, then it is not necessary to further discuss it in subsequent appended drawings.

In this document, vectors are a set of various variables provided by a patient. Model f is a mapping function: $X \rightarrow \{0, 1, 2, ...., n\}$, n may be, for instance, 3,4 or other integers. That is, if the index vector x of a patient is given, the model predicts that the pathological staging of hepatic fibrosis of this patient is $f(x)$, the value of which is one of the n discrete values in the set {0, 1, 2, ...., n}. Specific variables and classification model are important contents of the technology. The variables and classification model used in this patent are illustrated as follows.

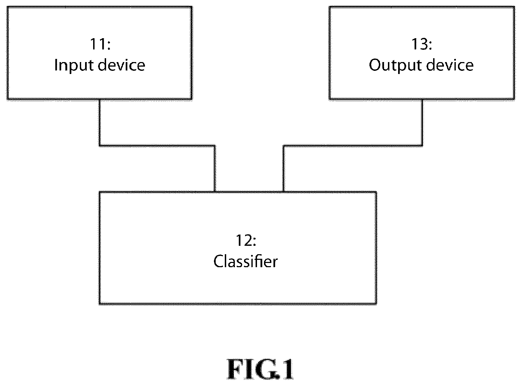

Figure 1 shows the structural diagram of a first embodiment of the hepatic fibrosis detection apparatus according to the present invention. As shown in Figure 1, the hepatic fibrosis detection apparatus in this embodiment comprises an input device 11, a classifier 12 and an output device 13. Wherein, the input device 11 is used to receive age and serum biochemical variables, and the serum biochemical variables include at least the blood platelet, hyaluronic acid (HA), serum direct bilirubin (DBIL), prothrombin time (PT), serum glutamic pyruvic transaminase (ALT; GPT) and serum glutamic oxaloacetic transaminase (AST; GOT). The classifier 12 performs hepatic fibrosis staging according to the age and serum biochemical variables received by the input device 11, and sends the hepatic fibrosis staging result to the output device 13. According to the three received variables, namely blood platelet, serum glutamic pyruvic transaminase (ALT; GPT) and serum glutamic oxaloacetic transaminase (AST; GOT), the classifier 12 obtains the ratio introduced by two experts: serum glutamic oxaloacetic transaminase (AST; GOT)/blood platelet and serum glutamic oxaloacetic transaminase (AST; GOT)/serum glutamic pyruvic transaminase (ALT; GPT), which are used to replace the serum glutamic oxaloacetic transaminase (AST; GOT) and serum glutamic pyruvic transaminase (ALT; GPT) as input parameters of the classifier. The output device 13 outputs the hepatic fibrosis staging results of the classifier 12. The classifier 12 may be a support vector machine classifier, a classifier based on the decision maker model, a support vector regression model classifier, a logistic regression classifier, an Adaboost ensemble classifier, or a PCA (principal component analysis)+KNN (K nearest neighbor) model classifier. The classifier 12 may be realized on a computing device through software, or be realized through special hardware, circuit or device.

In the above embodiment, the classifier can be used to obtain more accurate hepatic fibrosis detection effect through age and selected serum biochemical variables than the detection method of the prior art. Detection of the serum biochemical variables, such as blood platelet, hyaluronic acid (HA), serum direct bilirubin (DBIL), prothrombin time (PT), serum glutamic pyruvic transaminase (ALT; GPT) and serum glutamic oxaloacetic transaminase (AST; GOT) is more popularized, and can be achieved in general hospitals. Therefore, the application and popularization of the scheme can be expanded, so as to reduce the overall cost and difficulty of the detection. In addition, different classifiers may be selected according to the actual needs, thereby increasing the accuracy of the classifier in practice.

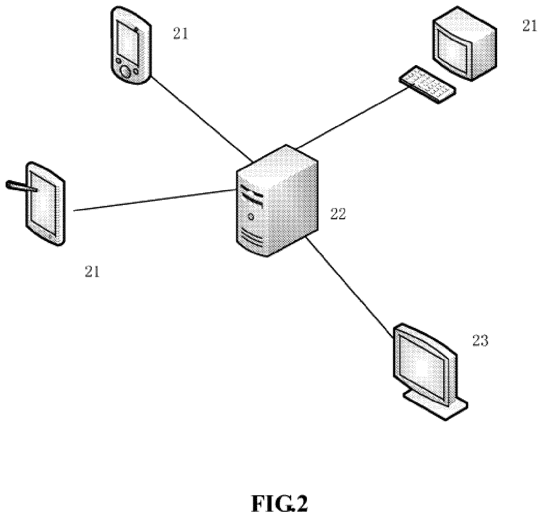

The hepatic fibrosis detection apparatus in the present invention may be realized in multiple forms according to the clinical needs. According to one embodiment of the present invention, the input device, classifier and output device are arranged in a computer, the input device and the output device correspond to the input equipment such as the computer keyboard, touch screen, mouse and device interface, and the output equipment such as the display screen, audio output device and output interface etc.; the classifier can be realized through software, or be realized through special classifier circuit connected to the motherboard. This detection apparatus can be achieved through a computer, and its implementation cost can be reduced by making full use of the characteristics of high popularization rate of computers. According to another embodiment of the present invention, the input device, classifier and output device are arranged in the same portable handheld device, which may be a general handheld computer, or a special device for diagnosis of hepatic fibrosis. The detection apparatus is realized in the form of a handheld device, which improves the convenience and flexibility for use of the device. According to one embodiment of the present invention, the hepatic fibrosis detection apparatus can also be achieved in the form of an online diagnostic system. A specific embodiment of an online diagnostic system is illustrated below with reference to Figure 2.

According to an embodiment of the present invention, the serum biochemical variables further include serum alkaline phosphatase (ALP; AKP), serum cholinesterase (ChE) and prothrombin activity (PTA), or any one or two of the above 3 variables.

According to an embodiment of the present invention, the serum biochemical variables also include the transforming growth factor $\beta 1$ (TGF-$\beta 1$) and $\alpha$ 2-macroglobulin (AMG); the classifier is used for hepatic fibrosis staging according to the received age and serum biochemical variables of the input device.

In the above embodiment, the classifier can be used to obtain more accurate hepatic fibrosis detection effect through age and selected serum biochemical variables than the detection method of the prior art.

Figure 2 shows the structural diagram of a second embodiment of the hepatic fibrosis detection apparatus according to the present invention. As shown in Figure 2, the input device 21 may be a computer, a tablet PC, or a PDA, etc. Equipment as the input device may be connected to the classifier 22 through wire connection or wireless connection etc. The classifier 22 may be a server, a computer or special equipment. The hepatic fibrosis staging result output by the classifier 22 may be output through the output device 23, or be output to the users through the input device 21. The detection apparatus can be realized in the form of an online diagnostic system only by a classifier in the background, which may include a plurality of input terminals and output terminals, so as to achieve detection support by more diagnosis sectors, and reduce the unit detection cost. According to an embodiment of the present invention, the input data of the classifier may include not only age and serum biochemical variables mentioned in above embodiment, but also transient hepatic elasticity imaging data of the liver tissue, namely the liver tissue stiffness values obtained through the transient elastography imaging apparatus.

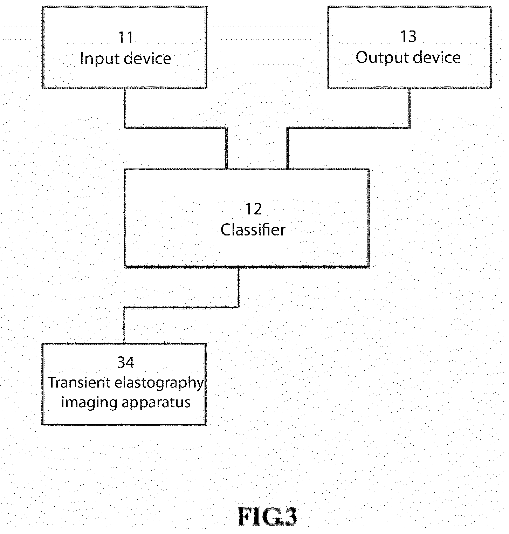

Figure 3 shows the structural diagram of a third embodiment of the hepatic fibrosis detection system according to the present invention. As shown in Figure 3, hepatic fibrosis detection system in this embodiment comprises an input device 31, a classifier 32, an output device 33 and a transient elastography imaging apparatus 34. Please refer to the description of the above embodiments for the input device 31 and output device 33, which are not illustrated in detail here for simplicity. Transient elastography imaging apparatus 34 can be used to obtain transient elastography imaging data of the liver tissue; the classifier 33 receives transient elastography imaging data of the liver tissue from the transient elastography imaging apparatus 34, and performs hepatic fibrosis staging based on age, serum biochemical variables and transient elastography imaging data of the liver tissue. Transient elastography imaging apparatus 34, FibroScan for instance, can be used to obtain FibroScan stiffness value of the liver tissue.

In the above embodiments, the system performs hepatic fibrosis staging in the light of the age, selected serum biochemical variables and transient elastography imaging data of the liver tissue, and makes full use of various detection results, so that the hepatic fibrosis staging results are more accurate.

According to one embodiment of the present invention, the system further comprises a serum biochemical variable detection apparatus, which is used to detect the samples in the kit, obtain the data of serum biochemical variables, and send such data to the classifier through the input device.

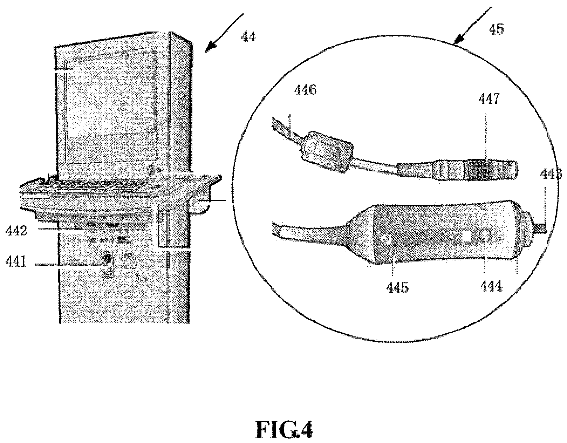

Figure 4 shows the schematic view of an embodiment of the transient elastography imaging apparatus and the probe thereof. As shown in Figure 4, the elasticity imaging apparatus 44 comprises a probe socket 441, which is used to connect with an ultrasound probe 45, and further comprises a data transmission interface 442, which is used to connect with a computer or network for data transfer. Ultrasound probe 45 comprises an ultrasound transducer 443, a switch button 444, an electrodynamics transducer 445, a connection cable 446 and a jack 447.

If there are fewer data samples of the training classifier parameters, over-fitting problem is very likely to arise only depending on a single model. Even if very favorable accuracy of the existing samples can be obtained, it may not have good generalization performance, and is difficult to correctly predict unknown samples.

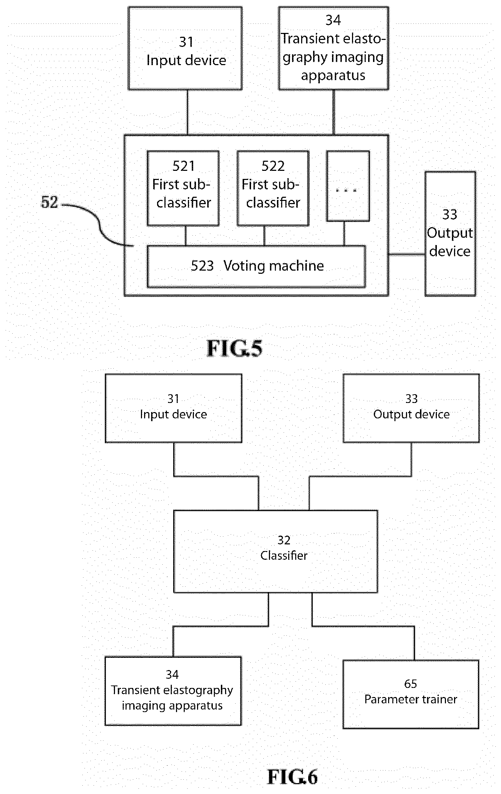

In order to solve this problem, Bagging method may be used: train a plurality of independent classifiers, and obtain the final classification result through voting as per the results of a plurality of classifiers. In this way, the non-robustness of prediction with only a single model can be solved to a certain extent. Completely different from the traditional bagging method, this Bagging method uses a method similar to cross-validation, randomly divides the samples into n aliquots each time, trains the classifier with n-1 portions thereof (parameters are also determined through the grid search method at this time), and predicts according to the remaining portion. Thus, screen the model according to the prediction results. By repeating a number of times of such random division, a certain model can be selected by random division every time. Finally, all obtained models are combined together to determine the final classification results by voting. Figure 5 shows the structural diagram of a fourth embodiment of the hepatic fibrosis detection system according to the present invention. As shown in Figure 5, the hepatic fibrosis detection system in this embodiment comprises an input device 31, a classifier 52, an output device 33 and a transient elastography imaging apparatus 34. Wherein, the input device 31, output device 33 and transient elastography imaging apparatus 34 can be found in the description of the above embodiments, and are not illustrated in detail here for simplicity. The classifier 52 comprises a voting machine 523, and two or more sub-classifiers such as the first sub-classifier 521, the second sub-classifier 522, and so on. Each sub-classifier 521, 522, etc. obtains their respective hepatic fibrosis staging results according to the age, serum biochemical variables and transient elastography imaging data of the liver tissue, and outputs their hepatic fibrosis staging results to the voting machine 523. The voting machine 523 determines the output hepatic fibrosis staging results according to the hepatic fibrosis staging results of each sub-classifier in the form of voting, for instance.

Figure 6 shows the structural diagram of a fifth embodiment of the hepatic fibrosis detection apparatus according to the present invention. As shown in Figure 6, the hepatic fibrosis detection apparatus in this embodiment comprises an input device 31, a classifier 32, an output device 33, a transient elastography imaging apparatus 34 and a parameter trainer 65. The parameter trainer 65 receives the training sample data, and determines the classifier parameters according to the training sample data; wherein, the training sample data may include age, serum biochemical variables and corresponding hepatic fibrosis staging; Or, the training sample data may include age, serum biochemical variables, transient elastography imaging data of the liver tissue and corresponding hepatic fibrosis staging. According to the existing sample, the hepatic fibrosis classification model can be obtained through training. Taking into account that the sample may be unceasingly enriched, therefore, a self-learning strategy of the model is designed. The learning strategy of the above model is completely compiled to an automated training process, the input interface is the sample set; and the output interface is the finally used prediction function. Therefore, once the sample set is updated, it is only necessary to adopt automatic training function of the program, so that the self-learning process of the model can be completed. Meanwhile, the old model will also be backed up and saved accordingly, so as to deal with the model restoration work under unexpected conditions. The classification model is introduced in the light of specific examples of support vector machines as follows. The training strategy of this classification model will be illustrated in detail below; relevant eigenvectors, if any, will be uniformly expressed as the vector $\chi$.

1. "Breakdown-combination" strategy of the model Breakdown

An original problem is to predict the stiffness value of a sample. It is more complex to directly solve this problem. First of all, the classification problem is broken down into four sub-problems:

```
Sub-Probleml:  S > = 1  vs S < 1

SubProblem2:   S >=2    vs S < 2

SubProblem3:   S >=3    vs S < 3

SubProblem4:   S >=4    vs S < 4
```

$$(1)$$

For example, sub-problem 1 means to determine whether the stiffness value of a given sample is greater than, equal to, or less than 1. This also applies to the remaining sub-problems.

Each sub-problem (binary classification problem) is trained using the support vector machine (SVM) classification model. Finally, a total of four sub-models $f_i(x)$, $i$=1, 2, 3, 4 are studied. The output of $f_i(x)$ is 0 or 1.

Please see the next section for detailed description of the support vector machine.

Combination

After completing the above four sub-problem models, the sub-problems can be combined into the final decision making rules. The results predicted with four sub-models are a sequence (*f1, f2, f3, f4*), every element in the sequence is 0 or 1, so there are a total of 16 possible values of the sequence. The decision is made according to the final prediction results corresponding to each value and the rules in Table 2.

**Four sub-models Final prediction results**

Table 1: Rules for combination of sub-models

| S>=1 | S>=2 | S>=3 | S>=4 | Predicted S |
|---|---|---|---|---|
| 0 | 0 | 0 | 0 | 0 |
| 0 | 0 | 0 | 1 | 0 |
| 0 | 0 | 1 | 0 | 0 |
| 0 | 0 | 1 | 1 | 3 |
| 0 | 1 | 0 | 0 | 2 |
| 0 | 1 | 0 | 1 | 4 |
| 0 | 1 | 1 | 0 | 3 |
| 0 | 1 | 1 | 1 | 4 |
| 1 | 0 | 0 | 0 | 1 |
| 1 | 0 | 0 | 1 | 2 |
| 1 | 0 | 1 | 0 | 3 |
| 1 | 0 | 1 | 1 | 4 |
| 1 | 1 | 0 | 0 | 2 |
| 1 | 1 | 0 | 1 | 4 |
| 1 | 1 | 1 | 0 | 3 |

(continued)

| S>=1 | S>=2 | S>=3 | S>=4 | Predicted S |
|---|---|---|---|---|
| 1 | 1 | 1 | 1 | 4 |

2. Support Vector Machine (SVM) classification model

As mentioned above, each model is divided into four sub-models, and each sub-model is a binary classification problem. The support vector machine is used as the basic classifier in this invention.

The support vector machine is an excellent classification model, which classifies the samples in the sample space according to the classification margin maximization principle, and ensures better generalization performance (the ability to predict unknown samples) on the premise of obtaining lower training error rate.

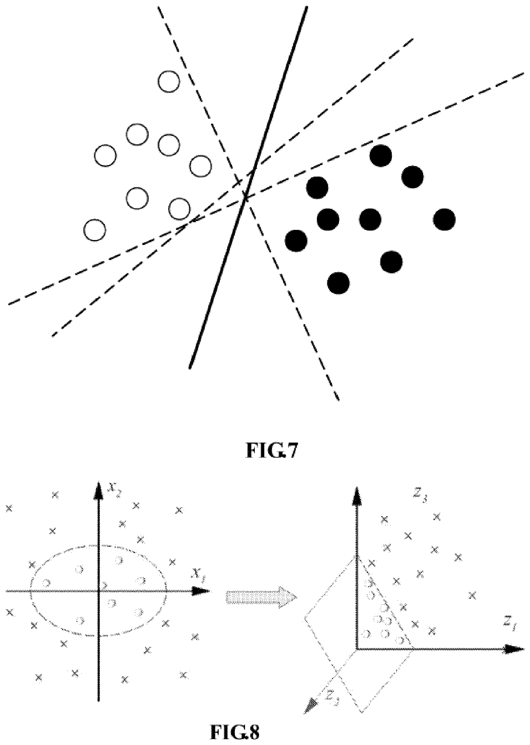

Figure 7 shows the schematic view of a linearly separable SVM classifier.

Figure 7: Schematic view of a maximum margin SVM classification hyperplane. Solid points and hollow points represent two types of sample points. The classification hyperplane of the intermediate solid line has larger classification margin than all remaining classification hyperplanes of dotted line, and has better generalization performance as a consequence.

Linear SVM

In simple terms, SVM is a linear classifier. For a binary classification problem, the training data set $\{(x_i, y_i)\} |n_{i=1}$ is given, where $x_i \in R^d, i = 1,2,..n$, n is an eigenvector, $y_i \in \{+1,-1\}$, $i$= 1, 2, .. n is the sample label. The classification rules are: $\hat{y} = sign\{w^T x + b\}$. $\chi$ is the new sample to be classified, $\hat{y}$ is the classification results of the SVM classifier model. $Sign(X)$ is a sign function, when $\chi$>= 0, $sign(X)$=1; when $\chi$<0, $sign(x)$ =-1.

Here, two variables determining the classifier need to be trained from data, and are specifically obtained through the following equation:

$$(\mathbf{w}^*, b^*) = \arg \min_{(\mathbf{w},b)} \frac{1}{2} \|\mathbf{w}\|_2^2 + C \sum_{i=1}^{n} \xi_i$$

$$s.t. : \forall i, y_i(\mathbf{w}^T \mathbf{x}_i + b) \geq 1 - \xi_i, \xi_i \geq 0 \quad (2)$$

Where, C is a parameter weighing the training error rate and generalization performance, and is usually determined through cross-validation.

In fact, the optimization problems determined through the equation 1 can be converted into the following dual problem:

$$\alpha^* = \arg \max_{\alpha} \alpha^T \mathbf{e} - \frac{1}{2} \alpha^T D\alpha$$

$$s.t. : 0 \leq \alpha \leq C, \alpha^T \mathbf{y} = 0 \quad (3)$$

Where, $\alpha = [\alpha_1,...,\alpha_n]^T, y = [y_1,...,y_n]^T, D = (D_{ij}), D_{ij} = y_i y_j x_i^T x_j$.

After the value of the dual variable $\alpha$ is obtained through solving the dual problem, the solution (w, b) of the original problem can be directly obtained as follows: $w = \sum_{i=1}^{n} \alpha_i y_i x_i$ · Therefore, the final classifier can be ex-

pressed as $\hat{y} = sign\{\sum_{i=1}^{n} \alpha_i y_i x_i^T x + b\}$ .

Nonlinear SVM

SVM can also learn a nonlinear model. It maps a sample from the original space into a higher dimensional feature space using the kernel method and a specific non-linear mapping, so that the linearly inseparable data in the original space can be linearly separable in the high-dimensional space. Thus, a linear model is designed in the high-dimensional space, and it is equivalent to a nonlinear model designed in the original space. Figure 8 shows a schematic view of improving a two-dimensional sample to a three-dimensional space through the polynomial kernel function, so that the original inseparable samples are linearly separable in a high-dimensional space. Figure 8 shows the schematic view of the nonlinear SVM algorithm. The original sample is linearly inseparable, and is converted through the following formula:

$$\Phi : R^2 \to R^3$$

$$(x_1, x_2) \mapsto (z_1, z_2, z_3) := (x_1^2, \sqrt{2}x_1 x_2, x_2^2) \qquad (4)$$

The original method is improved to a high-dimensional space using the kernel method, so that it is linearly separable in the high-dimensional space, which is equivalent to being nonlinearly separable in the original space.

As can be seen from the above linear SVM, either the dual form of SVM or final solution of classifier can be expressed as the inner product $x_i^T x_j$ of samples. Therefore, the kernel method is used for nonlinear mapping of samples $\Phi:x\to\Phi(\chi)$. In this way, in the high-dimensional space after mapping, the inner product between samples can be very easily calculated: $\Phi(x_i)^T\Phi(x_j) = K(x_i,x_j)$. K is the kernel function, such as the Gaussian kernel function:

$$K(x_i, x_j) = \exp(-\frac{(x_i - x_j)^2}{2\sigma^2}) \qquad (5)$$

Therefore, the nonlinear SVM classifier can be expressed as $\hat{y} = sign\{\sum_{i=1}^{n} \alpha_i y_i K(x_i, x_j) + b\}$ .

Where, the dual variables can be obtained through solving the dual problem 6:

$$\alpha^* = \arg\max_{\alpha} \alpha^T \mathbf{e} - \frac{1}{2}\alpha^T D^K \alpha$$

$$s.t. : 0 \leq \alpha \leq C, \ \alpha^T \mathbf{y} = 0 \qquad (6)$$

Where, $\alpha = [\alpha_1,...,\alpha_n]^T, e = [1,....,1]^T, y = [y_1,...,y_n]^T, D^K = (D_{ij}^K), D_{ij}^K = y_i y_j K(x_i, x_j)$ .

Generally, the kernel function needs to satisfy Mercer conditions. There are three frequently seen kernel functions:

1) Polynomial kernel function: $K(x_i, x_j)=(x^T y+c)^p, c \in R$

2) Gaussian kernel function: $K(x_i, x_j) = \exp(-(x_i - x_j)^2/(2\sigma^2))$

3) Sigmoid kernel function: $K(x_i, x_j) = \tanh(kx^T y - \delta)$

The nonlinear SVM can be used as the most basic classifier, and the Gaussian kernel is selected as the kernel.

The above biochemical variables and model obtain preferred parameters. In fact, with the above strategy, several other sets of parameters are also additionally obtained:

1. Variable parameters

Table 2

| Age and 9 serum biochemical variables | 1, 2, 3, 4, 5, 6, 7, 8, 9, 10 |
|---|---|
| Age, 9 serum biochemical variables and FibroScan stiffness value | 1, 2, 3, 4, 5, 6, 7, 8, 9, 10, 13 |
| Age and 11 serum biochemical variables | 1, 2, 3, 4, 5, 6, 7, 8, 9, 10, 11, 12 |
| Age, 11 serum biochemical variables and FibroScan stiffness value | 1, 2, 3, 4, 5, 6, 7, 8, 9, 10, 11, 12, 13 |

Medical meaning of the characteristic number used in the above models is indicated as follows:

Table 3

| Characterist ic number | Medical name | Remarks |
|---|---|---|
| 1 | Age | Age |
| 2 | Blood platelet | Serum biochemical variable |
| 3 | Serum alkaline phosphatase (ALP; AKP) | Serum biochemical variable |
| 4 | Serum cholinesterase (ChE) | Serum biochemical variable |
| 5 | Serum glutamic oxaloacetic transaminase (AST; GOT)/serum glutamic pyruvic transaminase (ALT; GPT) | Specific value introduced by experts (14/15) |
| 6 | Hyaluronic acid (HA) | Serum biochemical variable |
| 7 | serum direct bilirubin (DBIL) | Serum biochemical variable |
| 8 | Serum glutamic oxaloacetic transaminase (AST; GOT)/blood platelet | Specific value introduced by experts (14/2) |
| 9 | Prothrombin activity (PTA) | Serum biochemical variable |
| 10 | Prothrombin time (PT) | Serum biochemical variable |
| 11 | Transforming growth factor $\beta1$ (TGF-$\beta1$) | Serum biochemical variable |
| 12 | $\alpha2$-macroglobulin (AMG) | Serum biochemical variable |
| 13 | FibroScan stiffness value | Transient elastography imaging data |

The above characteristics 5 and 8 are 2 specific value characteristics introduced according to the expert advice. They are related to three characteristics 2, 14 and 15. The characteristic 2 is provided in the above table, and the characteristics 14 and 15 are as follows:

Table 4

| Characteri stic number | Medical name | Remarks |
|---|---|---|
| 14 | Serum glutamic oxaloacetic transaminase (AST; GOT) | Serum biochemical variable |
| 15 | Serum glutamic pyruvic transaminase (ALT; GPT) | Serum biochemical variable |

Table 5

| Age and serum biochemical variable | 1, 2, 3, 4, 5, 6, 7, 8, 9, 10 |
|---|---|
| Age, serum biochemical variable and FibroScan stiffness value | 1, 2, 3, 4, 5, 6, 7, 8, 9, 10, 13 |
| Age and serum biochemical variable | 1, 2, 3, 4, 5, 6, 7, 8, 9, 10, 11, 12 |
| Age, serum biochemical variable and FibroScan stiffness value | 1, 2, 3, 4, 5, 6, 7, 8, 9, 10, 11, 12, 13 |

Table 6

| Age and serum biochemical variable | 1, 2, 3, 4, 5, 6, 7, 8, 9, 10 |
|---|---|
| Age, serum biochemical variable and FibroScan stiffness value | 1, 2, 3, 4, 5, 6, 7, 8, 9, 10, 13 |
| Age and serum biochemical variable | 1, 2, 3, 4, 5, 6, 7, 8, 9, 10, 11, 12 |
| Age, serum biochemical variable and FibroScan stiffness value | 1, 2, 3, 4, 5, 6, 7, 8, 9, 10, 11, 12, 13 |

Table 7

| Age and serum biochemical variable | 1, 2, 3, 4, 5, 6, 7, 8, 9, 10 |
|---|---|
| Age, serum biochemical variable and FibroScan stiffness value | 1, 2, 3, 4, 5, 6, 7, 8, 9, 10, 13 |
| Age and serum biochemical variable | 1, 2, 3, 4, 5, 6, 7, 8, 9, 10, 11, 12 |
| Age, serum biochemical variable and FibroScan stiffness value | 1, 2, 3, 4, 5, 6, 7, 8, 9, 10, 11, 12, 13 |

Note that the characteristics are arranged in random order. Various models are related to 13 different characteristics, which are divided into three types: age, serum biochemical variables and FibroScan stiffness value. The serum biochemical variables include the blood platelet, hyaluronic acid (HA), serum direct bilirubin (DBIL), prothrombin time (PT), serum glutamic pyruvic transaminase (ALT; GPT), serum glutamic oxaloacetic transaminase (AST; GOT), transforming growth factor$\beta$1 (TGF-$\beta$1), and $\alpha$2-macroglobulin (AMG). The cost required to obtain them is different.
With different characteristics, different models can be obtained through training.

1) The model 1 uses the fewest characteristics, which are all biochemical variable characteristics that are frequently used in detection and can be achieved in general hospitals, and therefore the model 1 is the simplest;
2) The model 4 uses all characteristics, and has the highest precision, but the adopted characteristics are related to the FibroScan stiffness value, serum biochemical variables, transforming growth factor $\beta$1 (TGF-$\beta$1), and $\alpha$ 2-macroglobulin (AMG). Therefore, the characteristics need to be collected at high cost;
3) Model 2 and Model 3 adopt corresponding tradeoff strategy with comprehensive consideration of the model precision and cost required to collect the characteristics, and are two compromise solutions.

The following Table 8 shows the test results of above 4 models:

Table 8

| | Model 1 | | Model 2 | |
|---|---|---|---|---|
| | Uniform accuracy | AUROC | Uniform accuracy | AUROC |
| S>=1 vs S<1 | 0.922447 | 0.887332 | 0.923596 | 0.951516 |
| S>=2 VS S<2 | 0.719892 | 0.790301 | 0.784346 | 0.857179 |
| S>=3 VS S<3 | 0.779190 | 0.837752 | 0.867289 | 0.914025 |
| S>=4 VS S<4 | 0.874367 | 0.931671 | 0.901842 | 0.930822 |
| | Model 3 | | Model 4 | |
| | Uniform accuracy | AUROC | Uniform accuracy | AUROC |
| S>=1 VS S<1 | 0.928795 | 0.995746 | 0.932477 | 0.998524 |

(continued)

|  | Model 3 | | Model 4 | |
|---|---|---|---|---|
|  | Uniform accuracy | AUROC | Uniform accuracy | AUROC |
| S>=2 VS S<2 | 0.773575 | 0.896643 | 0.809324 | 0.924765 |
| S>=3 VS S<3 | 0.800767 | 0.887719 | 0.871436 | 0.932704 |
| S>=4 VS S<4 | 0.890920 | 0.929467 | 0.917976 | 0.945950 |

In the embodiments of the present invention, a classification model is designed based on medical indicators such as serum biochemical variables and FibroScan variables according to the "gold standard", so as to non-invasively predict hepatic fibrosis staging. Eigenvectors of the patient condition are obtained through test of specific biochemical variables of the patients. Based on the eigenvectors, the model predicts current pathological staging S0-S4 (or F0-F4) of the patients (The higher the level is, the more severe the hepatic fibrosis is) .

The technical solution in the present invention is selected from a plurality of parameters, mainly including: gender, age, HBV DNA level, a variety of liver enzyme variables, related cholesterol, and almost all biochemical variables, special detection index of hepatic fibrosis, FibroScan stiffness value and so on. Through analysis, processing and calculation of all above parameters, n serum biochemical variables with the best correlation with hepatic fibrosis are ultimately determined for clinical diagnosis, and the model for diagnosis of hepatic fibrosis and hepatic cirrhosis is obtained in combination with the FS detection result. Taking into account that various hospitals have different devices and biochemical test levels, the model is also divided into two versions, in order to facilitate detection in different hospitals.

1) FS+ biochemical test model) : Used in special hospitals /clinics for liver disease. The variables cover FS stiffness value and serum biochemical variables, such as blood platelet, hyaluronic acid (HA), serum direct bilirubin (DBIL), prothrombin time (PT), serum glutamic pyruvic transaminase (ALT; GPT) and serum glutamic oxaloacetic transaminase (AST; GOT).

2) FS+ detection model for all relevant biochemical indictors): Used to deeply solve diagnosis problems in special hospitals/clinics for liver disease with detection apparatus and higher scientific research level. The variables cover serum biochemical variables, such as blood platelet, hyaluronic acid (HA), serum direct bilirubin (DBIL), prothrombin time (PT), serum glutamic pyruvic transaminase (ALT; GPT), serum glutamic oxaloacetic transaminase (AST; GOT), FS stiffness value, serum transforming growth factor $\beta1$ (TGF-$\beta1$) and $\alpha$ 2-macroglobulin (AMG).

The hepatic fibrosis detection system in the embodiments of the present invention is characterized by noninvasiveness, strong practicability, simple method, low price and good security etc.:

(1) No risks. According to noninvasive medical equipment FibroScan and related blood biochemical results, the diagnostic system can determine the degree of hepatic fibrosis in patients with liver disease through model analysis almost without any risks, and will not be invasive to the patients.

(2) Low comprehensive cost. The liver biopsy needs not only the blood test, but also paracentesis and post-traumatic treatment, so it needs higher comprehensive cost than non-invasive diagnostic methods.

(3) The method is simple with wide range of clinical applications. It takes shorter time for Fibroscan operators to obtain the certificate, and the method is simple and easy to operate. Detection of biochemical variables no longer needs special training, and the hospital itself has conditions; the non-invasive model combining both has wide range of clinical applications. The description in the present invention is provided for illustration and description, but is neither exhaustive nor intended to limit the present invention to the disclosures. Many modifications and variations are obvious for general technical personnel in this field. Selection and description of the embodiments is intended to better illustrate the principles and practical application of the present invention, and allows the general technical personnel in this field to understand the present invention, so as to design various embodiments with various modifications suitable for particular purpose.

**Claims**

1. An apparatus for detecting hepatic fibrosis, **characterized by** comprising:

An input device (11, 31), used to receive age, serum biochemical variables and transient elastography imaging data, wherein the serum biochemical variables at least includes blood platelet, hyaluronic acid (HA), serum direct bilirubin (DBIL), prothrombin time (PT), serum glutamic pyruvic transaminase (ALT; GPT), serum glutamic oxaloacetic transaminase (AST; GOT), serum alkaline phosphatase (ALP; AKP), serum cholinesterase (ChE), prothrombin activity (PTA), transforming growth factor $\beta 1$ (TGF- $\beta 1$) and $\alpha 2$-macroglobulin (AMG)and wherein the transient elastography imaging data at least includes FibroScan stiffness value;

A classifier (12, 32, 52), used for hepatic fibrosis staging or inflammation diagnosis according to the age, said serum biochemical variables and said transient elastography imaging data received by the input device (11, 31), wherein the classifier (12, 32, 52) is a linear support vector machine classifier (12, 32, 52) or a nonlinear support vector machine classifier (12, 32, 52) based on kernel method;

An output device (13, 33), used to output the said hepatic fibrosis staging or inflammation diagnosis results of the said classifier (12, 32, 52).

2. The apparatus defined in claim 1, **characterized in that**, the said classifier (12, 32, 52) comprises at least two different classifiers (12, 32, 52), and obtains the hepatic fibrosis staging according to the results of at least two of the above different classifiers (12, 32, 52).

3. The apparatus defined in claims 1-2, **characterized in that**, the apparatus further comprises a parameter trainer (65) used to receive training sample data, and determine the parameters of the said classifier (12, 32, 52) based on the said training sample data; wherein, the said training sample data include at least the said age, serum biochemical variables, transient elastography imaging data, and corresponding hepatic fibrosis staging.

4. The apparatus defined in claims 1 to 3, **characterized in that**, the said apparatus is realized in the form of a handheld device, an online diagnosis system, or a stand-alone computing device.

5. A hepatic fibrosis detection system, **characterized in that**, the system includes the hepatic fibrosis detection apparatus defined in any one of claims 1 to 4 and a transient elastography imaging apparatus (34); wherein, the said transient elastic imaging apparatus (34) is used to obtain transient elastography imaging data of the liver tissue; the said classifier (12, 32, 52) receives transient elastography imaging data of the liver tissue from the transient elastography imaging apparatus (34), and performs hepatic fibrosis staging according to the said age, the said serum biochemical variables and the said transient elastography imaging data of the liver tissue.

6. The system defined in claim 5, **characterized in that**, the system further comprises a serum biochemical variable detection apparatus, the said serum biochemical variable detection apparatus is connected to the said input device (11, 31), and sends the said detected serum biochemical variables to the said classifier (12, 32, 52) through the input device (11, 31).

**Patentansprüche**

1. Gerät zum Feststellen von Leberfibrose, **dadurch gekennzeichnet, dass** es umfasst:

eine Eingabevorrichtung (11, 31), die zum Empfangen des Alters, der biochemischen Serumvariablen und der transienten, bildgebenden Elastografiedaten verwendet ist, wobei die biochemischen Serumvariablen wenigstens Blutplättchen, Hyaluronsäure (HA), direktes Serumbilirubin (DBIL), eine Prothrombinzeit (PT), Serum-Glutamat-Pyruvat-Transaminase (ALT; GPT), Serum-Glutamat-Oxalacetat-Transaminase (AST; GOT), alkaline Serum-Phosphatase (ALP; AKP), Serum-Cholinesterase (ChE), eine Prothrombin-Aktivität (PTA), einen transformierenden Wachstumsfaktor $\beta 1$ (TGF-$\beta 1$) und $\alpha 2$-Makroglobulin (AMG) einschließen und wobei die transienten, bildgebenden Elastografiedaten wenigstens einen FibroScan-Steifigkeitswert einschließen;

einen Klassierer (12, 32, 52), der zum Bereitstellen von Leberfibrose oder zur Diagnose einer Entzündung nach dem Alter verwendet ist, wobei die genannten biochemischen Serumvariablen und die genannten transienten, bildgebenden Elastografiedaten durch die Eingabevorrichtung (11, 31) empfangen sind, wobei der Klassierer (12, 32, 52) ein linearer Trägervektor-Maschinenklassierer (12, 32, 52) oder ein nicht linearer Trägervektor-Maschinenklassierer (12, 32, 52) basierend auf dem Kernel-Verfahren ist;

eine Ausgabevorrichtung (13, 33), die zum Ausgeben der genannten Bereitstellung von Leberfibrose oder von Ergebnissen der Diagnose einer Entzündung des genannten Klassierers (12, 32, 52) verwendet ist.

2. Gerät gemäß Definition in Anspruch 1, **dadurch gekennzeichnet, dass** der genannte Klassierer (12, 32, 52) wenigstens zwei unterschiedliche Klassierer (12, 32, 52) umfasst und die Bereitstellung von Leberfibrose gemäß den Ergebnissen von wenigstens zwei der obigen unterschiedlichen Klassierern (12, 32, 52) erhält.

**3.** Gerät gemäß Definition in Anspruch 1 - 2, **dadurch gekennzeichnet, dass** das Gerät weiterhin einen Parameter-Trainer (65) umfasst, der zum Empfangen von Schulungs-Beispieldaten verwendet ist und die Parameter des genannten Klassierers (12, 32, 52) basierend auf den genannten Schulungs-Beispieldaten bestimmt; wobei die genannten Schulungs-Beispieldaten wenigstens das genannte Alter, die biochemischen Serumvariablen, die transienten, bildgebenden Elastografiedaten und die entsprechende Bereitstellung von Leberfibrose einschließen.

**4.** Gerät gemäß Definition in Anspruch 1 bis 3, **dadurch gekennzeichnet, dass** das genannte Gerät in Form von einer tragbaren Vorrichtung, eines Online-Diagnosesystems oder einer eigenständigen Berechnungsvorrichtung realisiert ist.

**5.** System zum Feststellen von Leberfibrose, **dadurch gekennzeichnet, dass** das System das Gerät zum Feststellen von Leberfibrose, das in irgendeinem der Ansprüche 1 bis 4 definiert ist, und ein transientes, bildgebendes Elastografiegerät (34) einschließt; wobei das genannte transiente, bildgebende, Elastografiegerät (34) verwendet ist, um transiente, bildgebende Elastografiedaten des Lebergewebes zu erhalten; der genannte Klassierer (12, 32, 52) transiente, bildgebende Elastografiedaten des Lebergewebes von dem transienten, bildgebenden Elastografiegerät (34) empfängt und die Bereitstellung von Leberfibrose nach dem genannten Alter, den genannten biochemischen Serumvariablen und die genannten transienten, bildgebenden Elastografiedaten des Lebergewebes durchführt.

**6.** System gemäß Definition in Anspruch 5, **dadurch gekennzeichnet, dass** das System weiterhin ein Gerät zum biochemischen, variablen Feststellen von Serum umfasst, das genannte Gerät zum biochemischen, variablen Feststellen von Serum an die genannte Eingabevorrichtung (11, 31) angeschlossen ist und die genannten biochemischen Serumvariablen durch die Eingabevorrichtung (11, 31) zum Klassierer (12, 32, 52) sendet.

## Revendications

**1.** Appareil pour détecter la fibrose hépatique, **caractérisé en ce qu'**il comprend :

- un dispositif d'entrée (11, 31) utilisé pour recevoir l'âge, les variables biochimiques sériques et les données d'imagerie d'élastographie transitoire, les variables biochimiques sériques comprenant au moins les plaquettes sanguines, l'acide hyaluronique (HA), la bilirubine directe sérique (DBIL), le temps de prothrombine (TP), la transaminase glutamique-pyruvique sérique (ALT ; GPT), la transaminase glutamiqueoxaloacétique sérique (AST ; GOT), la phosphatase alcaline sérique (ALP ; AKP), la cholinestérase sérique (ChE), l'activité de prothrombine (PTA), le facteur de croissance transformant $\beta 1$ (TGF-$\beta 1$) et l'$\alpha 2$-macroglobuline (AMG) et les données d'imagerie d'élastographie transitoire comprenant au moins une valeur de rigidité FibroScan ;
un classifieur (12, 32, 52) utilisé pour déterminer le degré de fibrose hépatique ou le diagnostic d'inflammation selon l'âge, lesdites variables biochimiques sériques et lesdites données d'imagerie d'élastographie transitoire étant reçues par le dispositif d'entrée (11, 31), le classifieur (12, 32, 52) étant un classifieur une machine à vecteurs de support linéaire (12, 32, 52) ou une machine à vecteurs de support non linéaire (12, 32, 52) basé sur une méthode à noyaux ;
un dispositif de sortie (13, 33) utilisé pour sortir lesdits résultats du degré de fibrose hépatique ou du diagnostic d'inflammation dudit classifieur (12, 32, 52).

**2.** Appareil défini dans la revendication 1, **caractérisé en ce que** ledit classifieur (12, 32, 52) comprend au moins deux classifieurs (12, 32, 52) différents et obtient le degré de fibrose hépatique selon les résultats d'au moins deux des classifieurs (12, 32, 52) différents ci-dessus.

**3.** Appareil défini dans les revendications 1-2, **caractérisé en ce que** l'appareil comprend en outre un dispositif d'entraînement de paramètres (65) utilisé pour recevoir des données d'échantillon d'entraînement et déterminer les paramètres dudit classifieur (12, 32, 52) basés sur lesdites données d'échantillon d'entraînement ; lesdites données d'échantillon d'entraînement comprennent au moins lesdits âge, variables biochimiques sériques, données d'imagerie d'élastographie transitoire et degré de fibrose hépatique correspondant.

**4.** Appareil défini dans les revendications 1 à 3, **caractérisé en ce que** ledit appareil est réalisé sous la forme d'un dispositif portable, d'un système de diagnostic en ligne ou d'un dispositif informatique autonome.

**5.** Système de détection de la fibrose hépatique **caractérisé en ce que** le système comprend l'appareil de détection de la fibrose hépatique défini dans l'une quelconque des revendications 1 à 4 et un appareil d'imagerie d'élastographie transitoire (34) ; ledit appareil d'imagerie d'élastographie transitoire (34) étant utilisé pour obtenir des données d'imagerie d'élastographie transitoire du tissu hépatique ; ledit classifieur (12, 32, 52) recevant les données d'imagerie d'élastographie transitoire du tissu hépatique provenant de l'appareil d'imagerie d'élastographie transitoire (34), et déterminant le degré de fibrose hépatique selon ledit âge, lesdites variables biochimiques sériques et lesdites données d'imagerie d'élastographie transitoire du tissu hépatique.

6. Système défini dans la revendication 5, **caractérisé en ce que** le système comprend en outre un appareil de détection des variables biochimiques sériques, ledit appareil de détection des variables biochimiques sériques étant connecté audit dispositif d'entrée (11, 31) et envoyant lesdites variables biochimiques sériques détectées audit classifieur (12, 32, 52) par le biais du dispositif d'entrée (11, 31).

| 11:<br>Input device | | 13:<br>Output device |
| --- | --- | --- |
| | 12:<br>Classifier | |

**FIG.1**

**FIG.2**

```
┌──────────────────┐            ┌──────────────────┐
│        11        │            │        13        │
│   Input device   │            │  Output device   │
└──────────────────┘            └──────────────────┘

          ┌────────────────────────────┐
          │             12             │
          │         Classifier         │
          └────────────────────────────┘

      ┌──────────────────────────┐
      │            34            │
      │  Transient elastography  │
      │     imaging apparatus    │
      └──────────────────────────┘
```

FIG.3

**FIG.4**

| 31<br>Input device | 34<br>Transient elasto-<br>graphy imaging<br>apparatus | | |
|---|---|---|---|

| | 521<br>First sub-<br>classifier | 522<br>First sub-<br>classifier | . . . | 33<br>Output<br>device |
|---|---|---|---|---|
| 52 | 523   Voting machine | | | |

**FIG.5**

| 31<br>Input device | 33<br>Output device |
|---|---|

| 32<br>Classifier |
|---|

| 34<br>Transient elastography<br>imaging apparatus | 65<br>Parameter trainer |
|---|---|

**FIG.6**

**FIG.7**

**FIG.8**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010106140 A1

- WO 2010097472 A1

**Non-patent literature cited in the description**

- Support Vector Machine-Based Feature Selection for Classification of Liver Fibrosis Grade in Chronic Hepatitis C. **ZHENG JIANG et al.** Journal of Medical Systems. Kluwer Academic Publishers-plenum Publishers, 12 September 2006, vol. 30, 389-394